(19) Europäisches Patentamt
European Patent Office
Office européen des brevets

(11) Publication number: 0 271 923 B1

# (12) EUROPEAN PATENT SPECIFICATION

(45) Date of publication of the patent specification: 22.08.90

(51) Int. Cl.⁵: C07C 275/54, A01N 47/34

(21) Application number: 87118843.9

(22) Date of filing: 18.12.87

(54) N-(2,6-difluorobenzoyl)-N'-3-chloro-4-[1,1,2-trifluoro-2-(trifluoromethoxy) ethoxy] phenyl urea having insecticidal activity.

(30) Priority: 19.12.86 IT 2276986

(43) Date of publication of application: 22.06.88 Bulletin 88/25

(45) Publication of the grant of the patent: 22.08.90 Bulletin 90/34

(84) Designated Contracting States: AT BE CH DE ES FR GB LI NL SE

(56) References cited:
EP-A- 0 071 279
EP-A- 0 194 688
EP-A- 0 203 618
EP-A- 0 219 460

CHEMICAL ATSTRACTS, vol. 102, no. 23, June 10, 1985, Columbus, Ohio, USA I. MATSUMUTO; "Insecticidal N-(2-chloro- or 2,6-difluorobenzoyl)-N'-(3,5-dichloro-4-alkoxyphenyl) ureas", pages 577-578, abstract-no. 203 749y & JP-A-59-212 463

(73) Proprietor: ISTITUTO GUIDO DONEGANI S.p.A., Via Caduti del Lavoro, I-28100 Novara(IT)

(72) Inventor: Massardo, Pietro, Dr.-Chem., 5, via Fra'Bartolomeo, I-20123 Milan(IT)
Inventor: Rama, Franco, Dr.-Chem., 1, via Rodi, I-21052 Busto Arsizio Varese(IT)
Inventor: Piccardi, Paolo, Dr.-Chem., 8, via E. De Marchi, I-20125 Milan(IT)
Inventor: Caprioli,Vincenzo,Dr.-Bio., 8, via Loriga, I-28028 S. Martino Siccomario Pavia(IT)

(74) Representative: Zumstein, Fritz jun., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The present invention relates to an insecticidal compound, and more precisely it relates to the compound: N-(2,6-difluorobenzoyl)-N'-3-chloro-4-[1,1,2-trifluoro-2-(trifluoromethoxy)ethoxy ] phenyl urea which is endowed with high insecticidal activity and is suitable for use in agrarian, forestry, civil and veterinary fields for fighting insect infestations.

In European Patent Application No. EPO 203,618 insecticidal compounds are described, derived from 1-benzoyl-3-arylureas, having the formula:

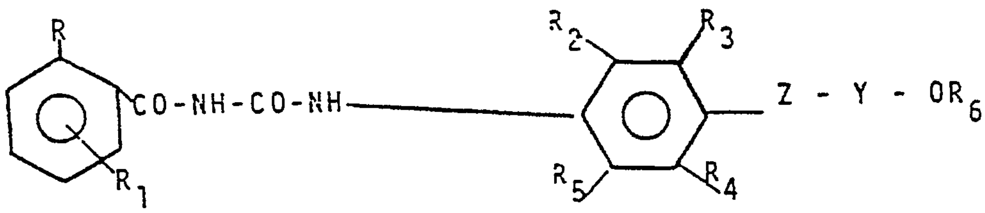

(I)

wherein:
R = Cl, F;
$R_1$ = H, Cl, F;
$R_2$ and $R_5$, which may be the same or different, are H, a halogen atom, a $C_1$-$C_4$ alkyl radical;
$R_3$ and $R_4$, which may be the same or different, are H, a halogen atom, an alkyl, haloalkyl, haloalkenyloxy, or alkynyl radical;
Z = O, S or a $NR_7$ group, wherein $R_7$ is a $C_1$-$C_3$ alkyl radical or H;
Y = a $C_1$-$C_4$ alkylene, haloethylene or haloethenyl; and
$R_6$ = a $C_1$-$C_4$ alkyl, $C_1$-$C_4$ haloalkyl, $C_3$-$C_4$ alkenyl, $C_3$-$C_4$ halo alkenyl, $C_3$-$C_4$ cycloalkyl, $C_3$-$C_4$ halocycloalkyl, or $C_3$-$C_4$ cycloalkenyl radical.

By examining the compounds disclosed in the above-mentioned European Patent application, it has now been discovered that, among the numerous compounds of formula (I), the particular compound N-(2,6-difluorobenzoyl)-N'-3-chloro-4-[ 1,1,2-trifluoro-2-(trifluoromethoxy)ethoxy ] phenyl urea is endowed, in a surprising way, with decisively higher insecticidal activity. Therefore the compound: N-(2,6-difluorobenzoyl)-N'-3-chloro-4-[1,1,2-trifluoro-2-(trifluoromethoxy)ethoxyl]phenylurea having the formula:

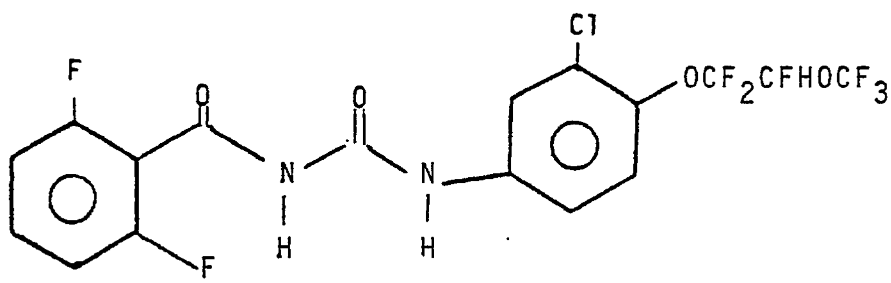

(II)

forms the major object of the present invention.

Compound (II) is endowed with an insecticidal activity that is particularly high and is unexpectedly higher than those of similar compounds of formula (I) disclosed in the above-mentioned European patent application.

The compound having formula (II) may be used either as such or in the form of suitable compositions for fighting infestations in noxious insects. Therefore the use of compound (II) for fighting insects and insecticidal compositions containing compound (II) as an active substance, form further objects of the present invention.

The preparation of compound (II) is carried out by employing the reaction, previously described in general in the above-mentioned European patent application, between a benzoyl-isocyanate and an aromatic amine; in particular, by reacting 2,6-difluorobenzoyl isocyanate (III) with 3-chloro-4-[ 1,1,2-trifluoro-2-(trifluoromethoxy)ethoxy] aniline (IV), according to the following reaction.

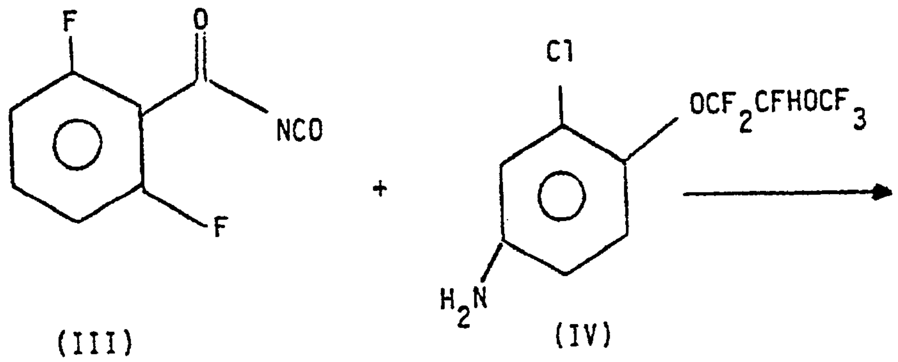

(III) (IV) (II)

The reaction does not require the presence of catalysts and is carried out in an inert solvent and at a temperature ranging from 0°C to the boiling temperature of the mixture.

The benzoylisocyanate of formula (III) is a known compound and may be prepared by known methods.

The amine having formula (IV) may be prepared by known methods and, more precisely, according to the methods described in the above-mentioned European patent application; preferably by reacting the sodium or potassium salt of 2-chloro-4-aminophenol (V) having the formula:

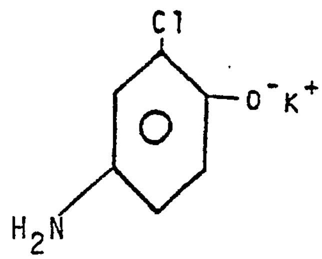

(V)

with perfluorovinyl-perfluoromethylether (VI) having the formula $CF_2{=}CF{-}OCF_3$, in dipolar aprotic solvents, at a temperature ranging from 0°C to room temperature.

An alternative process for the synthesis of the compound having formula (II) consists in reacting 2,6-difluorobenzamide (VII) with 3-chloro-4-[1,1,2-trifluoro-2-(trifluoromethoxy)ethoxy]phenyl isocyanate (VIII) according to the following scheme:

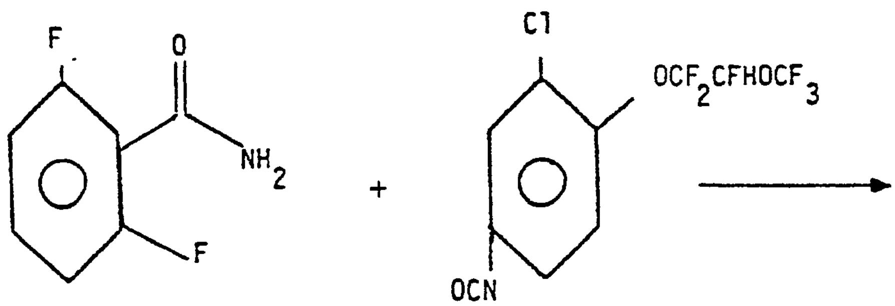

(VII) (VIII) (II)

Such reaction is carried out under conditions similar to those described for the reaction between benzoylisocyanate having the formula (III) with the amine having the formula (IV).

As above mentioned, N-(2,6-difluorobenzoyl)-N'-3-chloro-4-[1,1,2-trifluoro-2-(trifluoromethoxy)-ethoxy]phenyl urea (II) is endowed with high insecticidal activity, that proves to be especially effective against eggs and larvae of insects.

These insects, particularly those belonging to the orders Lepidoptera, Diptera, Coleoptera, may be successfully fought by using the compound having the formula (II).

These orders include several species which are important owing to their noxiousness in agrarian, forestry, civil and veterinary fields. Therefore N-(2,6-difluorobenzoyl)-N'-3- chloro-4-[1,1,2-trifluoro-2-(trifluoromethoxy)ethoxy]phenyl urea (II) proves to be suitable for several uses, such as, for instance, protection of agricultural cultivations against infestation of phytophagous insects, protection of environments infested with mosquitoes and flies, protection of breeding animals from certain cattle parasites, and the like.

In practical applications N-(2,6-difluorobenzoyl)-N'-3-chloro-4-[1,1,2-trifluoro-2-(trifluoromethoxy)-ethoxy]phenyl urea (II) may be used either as such or, more conveniently, in the form of compositions containing, besides the active substance, solid or liquid carriers and, optionally, other additives. The compositions may be readily formulated, according to conventional practice, in the form of wettable powders, emulsifiable concentrates, and the like.

The amount of N-(2,6-difluorobenzoyl)-N'-3-chloro-4-[1,1,2-trifluoro-2-(trifluoromethoxy)ethoxy]-phenyl urea (II) in the compositions ranges over wide limits (1-95% by weight), depending on the type of composition and on its intended use.

Furthermore, the amount of active substance to be distributed for the insecticidal treatment depends on different factors, such as for instance the kind of infestation, the environment wherein the infection breaks out (agrarian cultivations, basins and waterways, organic substrates of different nature), the type of composition that has been used, climatic and environmental factors, available application means, and the like. In general, amounts of active substance ranging from 0.01 and 1 Kg/ha are sufficient for good disinfestation.

The invention will now be still more fully illustrated by the following examples.

<u>Example 1</u>

Preparation of N-(2,6-difluorobenzoyl)-N'-3-chloro-4-[1,1,2-trifluoro-2-(trifluoromethoxy)ethoxy]-phenyl urea (II).

22.7 g of 3-chloro-4-[1,1,2-trifluoro-2-(trifluoromethoxy)ethoxy]aniline dissolved in 60 ml of anhydrous chlorobenzene were introduced into a three-neck flask having a capacity of 500 ml, equipped with a cooler, a thermometer, a dropping funnel, and a magnetic stirrer, in a nitrogen atmosphere.

Then, 13.4 g of 2,6-difluorobenzoyl-isocyanate dissolved in 40 ml of anhydrous chlorobenzene were dripped into the flask at room temperature. The mixture was heated, under stirring, for 12 hour at 100°C, then it was cooled to 0°C, filtered under nitrogen and the precipitate washed with cold n-hexane and finally dried under nitrogen.

30.5 g (84% of benzoylurea (II) were obtained having a melting point of 172°-174°C.

<u>Example 2</u>

Preparation of the 3-chloro-4-[1,1,2-trifluoro-2-(trifluoromethoxy)ethoxy]aniline starting material.

1.44 g of 2-chloro-4-amino phenol dissolved in 40 ml of a mixture consisting of toluene and dimethyl sulfoxide in a 1:1 ratio were loaded, under nitrogen, into a 3-neck flask having a capacity of 100 ml, equipped with a thermometer, a pierceable baffle for drawing off samples (e.g., a pierceable rubber plate), a connection to a gaseous reserve of perfluoro vinyl-perfluoromethyl ether, and a magnetic stirrer. After addition of 100 mg of finely ground KOH at 85%, the mixture was cooled to 0°C and the system was evacuated; 1.66 g of perfluoro-vinyl-perfluoromethyl ether were introduced from the gaseous reserve.

The whole was permitted to react at 0°C for 3.5 hours, then after having poured the reaction mixtue into 100 ml of water, one extracted with ethyl ether. One dehydrated over sodium sulfate and one concentrated, thereby obtaining 3.0 g of 3-chloro-4-[1,1,2-trifluoro-2-(trifluoromethoxy)ethoxy]aniline.

1H NMR 7.28-6.4 (m, 3H, arom); 6.3-5.69 (dt, 1H, -CFH-); 3.58(sb, 2H,-NH2).

<u>Example 3</u>

Determination of the insecticidal activity.

<u>Test 1</u>

Activity against larvae of <u>Spodoptera littoralis</u> (Lepidoptera).

Tobacco leaves were sprinkled mechanically with a water-acetone solution of N-(2,6-difluorobenzoyl)-N'-3-chloro-4-[1,1,2-trifluoro-2(trifluoromethoxy)ethoxy]phenyl urea (II) at 10% by volume of acetone and containing a surfactant.

After complete evaporation of the solvents, the leaves were infested with second age larvae of Lepidoptera.

The infested leaves were kept in a suitably conditioned environment over the time required for the test.

Tobacco leaves, treated only with a water-acetone solution at 10% of acetone and the surfactant, to be used as comparison (comparison test), were infested and kept likewise.

Ten days after the infestation and after having renewed the treated substrate at least once, the dead larvae were counted with respect to the results of the comparison test.

Test 2

Activity against larvae of Aedes aegypti (Diptera).

Spring-water (297 ml) was mixed with an acetone solution (3 ml) containing N-(2,6-difluorobenzoyl)N'-3-chloro-4-[1,1,2-trifluoro(2-trifluormethoxy)ethoxy]phenyl urea (II) in a suitable concentration.

25 larvae of Diptera being 4 days of age, fed suitably, were introduced into the obtained solution. Other larvae were introduced, as a comparison test, into a water-acetone solution (spring-water 297 ml, acetone 3 ml) without any active ingredient.

Every 2-3 days one took a note of the number of dead larvae and pupae and of adults emerged normally, till completion of the emergence of the insects in the comparison sample.

The activity of the product being tested was expressed as a per cent ratio of dead individuals, compared with the total number of treated individuals.

Test 3

Activity against Leptinotarsa decemlineata (Coleoptera).

Potato plants (about 10 cm high) were treated by immersion into a water-acetone dispersion of N-(2,6-difluorobenzoyl)-N'-3-chloro-4-[1,1,2-trifluoro-2-(trifluoromethoxy)ethoxy]phenyl urea (II) at 10% by volume of acetone and containing a surfactant.

After drying the deposit, the plants were infested with groups of 10 second age larvae of Coleoptera; afterwards they were kept apart suitably.

The material being tested was maintained in a conditioned environment and at continuous lighting over the time required for the test.

72-96 hours after the treatment, untreated plants were fed to all the larvae, in order to supply them with new food.

The activity, expressed as percentage of mortality, corrected with respect to the comparison test, was surveyed every 8 days after the beginning of the test, by reckoning the dead larvae and not completely live ones present in each sample (2 repetitions, each one consisting of a plant with 10 larvae).

The data of insecticidal activity (per cent mortality) are reported in the following Table 1) , at the indicated doses expressed in parts per million of active substance of compound (II) according to the invention, compared with four similar reference compounds (Ref. 1, Ref. 2, Ref. 3, Ref. 4) described in European patent application No. EPO 203,618 referred to above.

TABLE 1

INSECTICIDE ACTIVITY

| Compound | (II) | | REF. 1 | | REF. 2 | | REF. 3 | | REF. 4 | |
|---|---|---|---|---|---|---|---|---|---|---|
| | DOSE PPM | MORTALITY | DOSE PPM | MORTALITY | DOSE PPM | MORTALITY | DOSE PPM | MORTALITY | DOSE PPM | MORTALITY |
| | 0.1 | 100 | 1.0 | 100 | 0.5 | 100 | 1.0 | 100 | 0.1 | 100 |
| Test 1 | 0.05 | 96 | 0.5 | 70 | 0.1 | 72 | 0.5 | 95 | 0.05 | 93 |
| | 0.01 | 76 | 0.1 | 35 | 0.05 | 40 | 0.1 | 78 | 0.01 | 78 |
| | 0.005 | 40 | – | – | 0.01 | 20 | 0.05 | 40 | 0.005 | 31 |
| | 0.002 | 100 | 0.2 | 100 | 20 | 100 | 0.02 | 100 | 2.0 | 100 |
| Test 2 | 0.0002 | 70 | 0.02 | 25 | 2 | 93 | 0.002 | 86 | 0.2 | 77 |
| | 0.00002 | 62 | – | – | 0.2 | 56 | 0.0002 | 41 | 0.02 | 37 |
| | – | – | – | – | 0.02 | 27 | 0.00002 | 34 | 0.002 | 17 |
| | 5.0 | 100 | 50.0 | 100 | 5.0 | 95 | 100 | 100 | 1.0 | 100 |
| Test 3 | 1.0 | 85 | 10.0 | 97 | 1.0 | 57 | 10 | 95 | 0.5 | 97 |
| | 0.5 | 52 | 5.0 | 17 | 0.5 | 17 | 5 | 72 | 0.1 | 47 |
| | – | – | – | – | – | – | 1 | 5 | 0.05 | 9 |

In Table 1 as reference 1 use was made of the following compound: N(2,6-difluorobenzoyl)-N'-4-[1,1,2-trifluoro-2-(trifluoromethoxy)ethoxy]phenyl urea; as reference 2 use was made of the following compound: N-(2,6-difluorobenzoyl)-N'-3,5-dichloro-4-[1,1,2-trifluoro-2-(trifluoromethoxy)ethoxy]phenyl urea; as reference 3 use was made of the following compound: N-(2,6-difluorobenzoyl)-N'-4[1,1,2-trifluoro-2-(perfluoroethoxy)ethoxy]phenyl urea; as reference 4 use was made of the following compound: N-(2-chlorobenzoyl)-N'-3,5-dichloro-4-[1,1,2-trifluoro-2-(trifluoromethoxy)ethoxy]phenyl urea.

## Claims

1. The compound N-(2,6-difluorobenzoyl)-N'-3-chloro-4-[1,1,2-trifluoro-2-(trifluoromethoxy)ethoxy]-phenyl urea.

2. A process for the preparation of the compound according to Claim 1, consisting essentially in reacting, in an inert solvent and at a temperature ranging from 0°C and the boiling temperature of the reaction mixture, 2,6-difluorobenzoyl isocyanate with 3-chloro-4-[1,1,2-trifluoro-2-(trifluoromethoxy)ethoxy]-aniline.

3. A method of fighting infestations of noxious insects consisting essentially in distributing in the infestation area an effective amount of the compound according to Claim 1, either as such or in the form of a suitable composition.

4. Insecticidal compositions containing, as an active ingredient, the compound according to Claim 1, together with solid or liquid inert carriers, and, optionally, other additives.

## Patentansprüche

1. Die Verbindung N-(2,6-Difluorobenzoy.-N'-3-chlor-4-[1,1,2-trifluor-2-(trifluormethox.-ethoxy]-phenylharnstoff.

2. Verfahren zur Herstellung der Verbindung gemäß Anspruch 1, bestehend im wesentlichen aus der Umsetzung in einem inerten Lösungsmittel und bei einer Temperatur im Bereich von 0°C und der Siedetemperatur der Reaktionsmischung von 2,6-Difluorbenzoylisocyanat mit 3-Chlor-4-[1,1,2-trifluor-2-(trifluormethox.-ethox.-anilin.

3. Verfahren zur Bekämpfung eines Befalls schädlicher Insekten, im wesentlichen darin bestehend, daß man in dem befallenen Bereich eine wirksame Menge der Verbindung gemäß Anspruch 1 entweder als solcher oder in Form einer geeigneten Zusammensetzung verteilt.

4. Insektizide Zusammensetzung, enthaltend als wirksamen Bestandteil die Verbindung gemäß Anspruch 1, zusammen mit festen oder flüssigen inerten Trägern und gegebenenfalls anderen Additiven.

## Revendications

1. Le composé consistant en N-(2,6-difluorobenzoyl)-N'-3-chloro-4-[1,1,2-trifluoro-2-(trifluorométhoxy)éthoxyl]-phényl-urée.

2. Un procédé de préparation du composé selon la revendication 1, consistant essentiellement en la réaction, dans un solvant inerte et à une température comprise entre 0°C et le point d'ébullition du mélange réactionnel, de l'isocyanate de 2,6-difluorobenzoyle avec la 3-chloro-4-[1,1,2-trifluoro-2-(trifluorométhoxy)éthoxy]aniline.

3. Un procédé de lutte contre l'infestation par des insectes nuisibles, consistant essentiellement à traiter l'air infestée par une quantité efficace du composé selon la revendication 1, soit tel quel soit sous forme d'une composition adéquate.

4. Des compositions insecticides contenant en tant qu'ingrédient actif le composé selon la revendication 1, avec des supports inertes solides ou liquides et, éventuellement, d'autres additifs.